# EUROPEAN PATENT APPLICATION

(11) **EP 2 368 872 A1**
(43) Date of publication of application: **28.09.2011**
(21) Application number: 10425091.5
(22) Date of filing: 25.03.2010
(51) Int. Cl.: C07C 227/18, C07C 229/28

(54) **Process for the preparation of Gabapentin**

(71) Applicant: Serichim S.r.l., 33050 Torviscosa (IT)
(72) Inventor: Delogu, Pietro, 34100 Trieste (IT); Cordero, Lucia, 33052 Cervignano del Friuli (IT); De Rosa, Sabrina, 33052 Cervignano del Friuli (IT); Puntin, Giulio, 34070 Turriaco (IT); Velardi, Rosario, 33100 Udine (IT)
(74) Representative: Cattaneo, Elisabetta

(57) **Abstract**

The present patent application discloses a method for the preparation of [(1-aminomethyl)cyclohexyl]acetic acid (Gabapentin) by means of the Hofmann re-arrangement reaction and the extraction of the Gabapentin from the reaction mixture with a higher aliphatic alcohol, wherein the Hofmann reaction is conducted continuously, for the purpose of obtaining an active ingredient with a good yield and a quality suitable for use in the preparation of pharmaceutical products.

## Description

### Field of the invention

The present invention relates to a method for the preparation of [(1-aminomethyl)cyclohexyl]acetic acid (Gabapentin) by means of the Hofmann re-arrangement reaction and the extraction of Gabapentin in zwitterionic form, wherein at least the Hofmann reaction is obtained in continuous mode.

### State of the art

Gabapentin is a pharmaceutical active ingredient effective against partial epileptic seizures and neuropathic pain. Since the daily doses required are relatively high, even as high as several grams a day, the quantities produced annually worldwide are very considerable, in excess of 1000 tonnes. That is why there is a strong demand to develop highly-efficient and low-cost production processes, also given the need to contain the social costs of epilepsy treatment.

Several methods are known for the synthesis and purification of Gabapentin. Among the main synthetic strategies developed, the one based on the Hofmann re-arrangement of [1-(2-amino-2-oxoethyl)cyclohexyl]acetic acid seems to be the most widespread in industrial use and to afford the lowest production costs. This synthetic process was first mentioned in 1977, in the US patent 4,024,175, and was subsequently described in numerous other patents involving the use of a batch technology. It is clear from the detailed description of the preparation of Gabapentin based on this Hofmann re-arrangement reaction that a laborious management of the methods for bringing the reagents into contact and a careful control of the temperatures are needed to obtain high yields. In fact, the first problem consists in the exothermicity of the reaction, which makes temperature control troublesome and dependent on the scale on which the reaction is conducted.

For instance, the patent application WO 02/34709 gives a detailed description of the preparation of raw Gabapentin based on the Hofmann re-arrangement reaction starting from two distinct aqueous solutions of 1,1-cyclohexanediacetic acid monoamide/NaOH and sodium hypochlorite/NaOH, the latter being prepared under vacuum and stirring. The Hofmann re-arrangement reaction is then conducted by adding the first solution to the second in 2.5 to 4 hours under a slight nitrogen flow, keeping an internal temperature of -5°C. The reaction mixture is maintained between -3°C and +5°C for two hours and then slowly brought up to 20°C in 2 to 3 hours and maintained at a temperature of 20-25°C for a further hour.

Similarly, the patent application WO 03/089403, in example 1, step 1 discloses the preparation of crude Gabapentin by Hofmann re-arrangement that involves a solution of sodium hydroxide and hypochlorite at 0°C being mixed with an aqueous solution of [1-(2-amino-2-oxoethyl)cyclohexyl]acetic acid and NaOH at temperatures between 0°C and 10°C, and made to react for a period of approximately 2-3 hours.

Basically, these methods of synthesis demand low temperatures and relatively long reaction times. As a consequence, these procedures are difficult to implement, scarcely reproducible and troublesome in industrial applications. Moreover, sodium hypochlorite is available at concentrations no higher than 14-15% by weight. The reaction accordingly takes place in highly diluted conditions, with relatively high reaction volumes. Large reaction volumes and reaction times of several hours give rise to a low yield and to the need to use systems of considerable dimensions in order to achieve the annual output required for an active ingredient in such widespread use. Hence, it is generally felt the need to have a technology that, although it must treat diluted solutions, it can assure a high productivity potentially even using small-sized equipment.

Gabapentin is generally obtained from the crude reaction product in the form of a hydrochloride. In a subsequent operation, the hydrochloride is then treated in various ways to obtain Gabapentin in zwitterionic form, for instance by means of:
- a treatment with anion-exchange resins (US 4,024,175);
- a treatment with strong cation-exchange resins after liquid-liquid extraction with alcoholic solvents, particularly n-butanol, (WO 02/34709);
- precipitation from water to the isoelectric point (US 6,518,456);
- exchange of hydrochloric acid with an organic base, generally an amine, and precipitation of either the amine hydrochloride or zwitterionic Gabapentin, depending on the solubilities in the solvent used.

In relation to this last Gabapentin separation method, WO 08/106217, for example, describes a treatment with tributylamine, the hydrochloride of which remains in solution, while the Gabapentin in zwitterionic form precipitates and is filtered; US 7,393,974 uses dicyclohexylamine instead, the hydrochloride of which precipitates, leaving Gabapentin in solution in zwitterionic form.

In any case, Gabapentin is obtained from the crude reaction product in the form of a hydrochloride, not in its final form as a free amino acid in zwitterionic form.

The above-mentioned purification methods involve the use of complex procedures (such as treatments with ion exchange resins) or of additional reagents (such as tributylamine), that constitute a source of waste products or make it necessary to undertake laborious and uneconomical recovery procedures.

The patent application WO 08/106217 describes the extraction of Gabapentin hydrochloride using a continuous procedure, i.e. countercurrent liquid-liquid extraction, alternated with a series of batch procedures. The option fails to assure all the advantages intrinsic in the use of a continuous process, because the running of a continuous procedure isolated from the rest of the process can only afford limited advantages. As mentioned previously, moreover, this extraction gives rise to Gabapentin hydrochloride and demands laborious procedures downstream to convert the hydrochloride into the zwitterionic form required.

The preparation of amines in a continuous process by Hofmann re-arrangement has been described starting from cyclopropane carboxamide, or from aromatic carboxamides (EP 00393350, EP 1418168 and US 3,322,820). However, the procedures described have not been applied to aliphatic carboxamides, and they often involve complex operating sequences in order to manage the reaction temperature and the contact between the reagents.

The availability of a process for the synthesis of aliphatic amines, such as Gabapentin, that is straightforward to manage on an industrial scale and capable of providing a pharmaceutical grade end-product is consequently a need that is strongly felt in the field.

Using continuous reaction and extraction of Gabapentin from the crude reaction product directly in zwitterionic form, the present invention entails a Gabapentin production process with a high yield that demands no complex steps for its management on an industrial scale as regards the conditions for the synthesis of the active ingredient or its separation from the reaction mixture, which is obtained without the intermediate hydrochloride formation. Given the continuous nature of the process, it is also easy to automate, affording a considerable saving on production costs.

A further object of the present invention is a process capable of providing an active ingredient with a high yield and purity suitable for the preparation of pharmaceutical products.

### Summary

The present invention concerns a process for the production of Gabapentin based on the following sequence of operations:
a) the continuous preparation of [(1-aminomethyl)cyclohexyl]acetic acid (Gabapentin) by Hofmann re-arrangement achieved by mixing an aqueous solution of [1-(2-amino-2-oxoethyl)cyclohexyl]acetic acid (cyclohexane diacetic acid monoamide) and a hydroxide of an alkali metal with an aqueous solution of hypohalogenite and of a hydroxide of an alkali metal;
b) the extraction of Gabapentin in zwitterionic form with a higher aliphatic alcohol C₄-C₇ from the reaction mixture, consisting of the crude product of the Hofmann re-arrangement reaction, after its neutralisation with an acid.
Gabapentin is obtained from the extracted alcoholic solution by filtering and/or by crystallisation, after the solution has been anhydrified by azeotropically distilling the water.

### Detailed description of the invention

The process according to the present invention involves Gabapentin prepared as a result of the Hofmann re-arrangement of cyclohexane diacetic acid monoamide by means of a technology carried out in continuous mode, as schematically represented below: 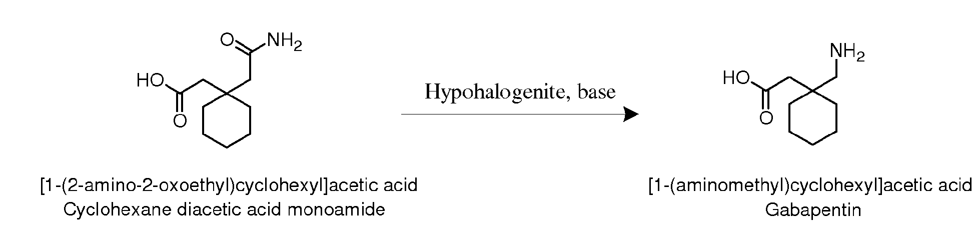 followed by liquid-liquid extraction with an aliphatic alcohol C₄-C₇ of Gabapentin in zwitterionic form from the crude reaction product and its subsequent separation from the alcoholic solution.

Thus, the method for the preparation of Gabapentin according to the invention comprises at least the following steps:
- mixing an alkaline aqueous solution of [1-(2-amino-2-oxoethyl)cyclohexyl]acetic acid with an alkaline aqueous solution of a hypohalogenite and making them react in a continuous reactor;
- extracting Gabapentin in zwitterionic form with a higher aliphatic alcohol C₄-C₇ from the mixture resulting from the Hofmann re-arrangement reaction, after its neutralisation with an acid.

Prior to extraction, the reaction mass deriving from the Hofmann re-arrangement is preferably treated with a reducing agent to reduce the excess hypohalogenite, then neutralised with a, preferably inorganic, acid.

Thus, in detail, the method for the preparation of Gabapentin according to the present invention comprises the following steps:
- preparing aqueous solutions of the reagents for the Hofmann re-arrangement reaction, consisting of an aqueous solution of [1-(2-amino-2-oxoethyl)cyclohexyl]acetic acid, obtained by dissolving the acid in an aqueous solution of an alkali, and an aqueous solution of a hypohalogenite and an alkali;
- mixing the alkaline aqueous solution of [1-(2-amino-2-oxoethyl)cyclohexyl]acetic acid with the alkaline aqueous solution of a hypohalogenite in a continuous reactor to perform the Hofmann reaction;
- treating the reaction mixture with a reducing agent to reduce the excess hypohalogenite contained in the crude reaction product coming from the reactor;
- neutralising the reaction mass to a pH in a range comprised from 6.0 to 7.5 by adding an acid;
- extracting Gabapentin in zwitterionic form from the crude reaction product with a higher aliphatic alcohol C₄-C₇.

Preferably the extraction is a continuous liquid-liquid process and more preferably is in counter-current mode.

This method, which is described below in detail, is particularly straightforward and suitable for the production of large quantities of active ingredient, achieving a high productivity, a low environmental impact and a reproducible quality. In fact, it has surprisingly been found that the Hofmann re-arrangement of the cyclohexane diacetic acid monoamide (i.e. [1-(2-amino-2-oxoethyl)cyclohexyl]acetic acid) can be achieved simply by delivering the monoamide and hypohalogenite solutions to a plug flow reactor in an alkaline environment. Optionally, the free carboxylic group of the [1-(2-amino-2-oxoethyl)cyclohexyl]acetic acid may be present as a carboxylate ion in salified form.

The reactor may be a tubular reactor or a cascade of agitated reactors that, as a person skilled in the art is well aware, equates to a plug flow reactor, or a combination of tubular and agitated sections. The reaction solvent can conveniently be water.

A number of hypohalogenites, e.g. hypobromite or sodium hypochlorite, can be used for the reaction. Sodium hypobromite can be generated on-site by adding bromide to an alkaline solution. However, sodium hypochlorite is preferred, given its commercial availability and ease of use.

The alkaline environment can be obtained by using any inorganic base and preferably from an alkali metal, selected from either sodium hydroxide or potassium hydroxide. Operating in an aqueous environment, sodium hydroxide is preferred.

The molar ratio between the hypohalogenite and the cyclohexane diacetic acid monoamide must be at least 1 mole of hypohalogenite per mole of monoamide. There is no upper limit to this ratio but it is advisable to keep it below 2 moles of hypochlorite per mole of cyclohexane diacetic acid monoamide for economic reasons and to minimise the quantity of salts generated by the process. In order to reduce to a minimum the quantity of hypochlorite used, it is advisable to control its concentration at the outlet from the reactor with the aid of a suitable analytical method. An adequate method consists in measuring the potential of the solution delivered, which must be oxidant.

The molar ratio between the alkali and the cyclohexane diacetic acid monoamide is determined by the stoichiometry of the Hofmann re-arrangement and may vary between 2 moles of alkali per mole of monoamide up to 4 moles of alkali per mole of monoamide. A ratio in the range comprised from 2.5 to 3.5 moles of alkali per mole of cyclohexane diacetic acid monoamide is preferable or, better still, a ratio in the range of 2.8 to 3.2.

The concentration of the reagents in the aqueous solvent is determined by the commercial availability of the raw materials and by the need to supply all the reagents in solution to avoid having to handle the feed of solids (although this is feasible, they are industrially more difficult to manage). The use of an aqueous solution of hypochlorite at concentrations in the range comprised from 4% to 15% is effective; a hypochlorite concentration between 10% and 14% is preferable. The retention times in the reactor system, calculated as the ratio of the reactor volume to the total flow rate of the reagents, may vary between 2 minutes and 180 minutes, and preferably between 5 minutes and 60 minutes or, better still, between 8 minutes and 20 minutes.

The temperature of the reactor can vary between 5°C and 90°C, and preferably between 10°C and 70°C or, better still, between 30°C and 50°C.

A reducing agent is added to the crude mixture coming from the reactor, produced according to the invention, in order to destroy the residual hypohalogenite, and the pH is adjusted with hydrochloric acid to the isoelectric point of Gabapentin, which is 7.2, in order to obtain the zwitterionic form in solution. These conditions can be conveniently achieved both in batch production modes and in continuous reactors; the continuous mode is the preferred. Suitable reducing agents are sodium or potassium sulphite, sodium metabisulphite, sodium thiosulphate and others known to a person skilled in the art.

The aqueous solution at a pH of 7.2 is treated according to the invention with a higher aliphatic alcohol C₄-C₇ to extract Gabapentin in the organic phase and separate it from the majority of the salts, which remain in the aqueous phase. This operation can also be conducted in a batch mode (by completing a series of extractions with several portions of alcohol), or in continuous mode (by feeding the crude reaction product and the extraction solvent into an extraction equipment). Said extraction equipment may be a countercurrent extraction column, such as the Scheibel, or Khuni, or other types known to a person skilled in the art, or it may be a series of mixer-settlers. The ratio between the flow rate of the alcohol and that of the neutralised reaction mixture, according to the preferred extraction conditions, is in the range of 0.5:1 to 3:1 v/v.

The method according to the present invention extracts Gabapentin directly in zwitterionic form in the organic phase, leaving the inorganic salts in the aqueous phase, and consequently producing only one saline waste product. The organic extract, which contains small residual quantities of salts, can be backwashed with salt-free water, recycling the saline aqueous solution in the previous extraction. All the extraction and counter-extraction steps can be conducted continuously in countercurrent mode.

Effective extraction solvents include the higher aliphatic alcohols C₄-C₇, selected from the group consisting of n-butyl alcohol, isobutyl alcohol, amyl alcohol, isoamyl alcohol, and mixtures thereof; n-butanol is preferred.

Gabapentin in zwitterionic form is obtained from the alcoholic solution by removing the water content by azeotropic distilling and then filtering the precipitated product. It has been established that removal of the water content in the alcoholic butanol solution substantially reduces the solubility of Gabapentin and enables the product to be recovered with a high yield.

The precipitated Gabapentin can be purified by recrystallising it from methanol, water-methanol mixtures or methanol-isopropanol mixtures of suitable composition, according to methods known to a person skilled in the art.

### Examples

### Example 1: Gabapentin synthesis in a reactor consisting of a series of agitated continuous reactors with a retention time of 30 minutes

A solution of 320 g of [1-(2-amino-2-oxoethyl)cyclohexyl]acetic acid in 644.7 g of 11 % aqueous sodium hydroxide and a solution of 786.11 g of 13.28% aqueous sodium hypochlorite and 46.99 g of 100% NaOH were fed into a reactor consisting of 10 agitated chambers in series providing a total free volume of 67.6 ml (Coflore ACR by AM Technology, http://www.amtechuk.com) and maintained at 40°C with flow rates of 0.68 ml/min and 1.53 ml/min, respectively, for a retention time of 30.6 minutes. Sodium metabisulphite (in 6% aqueous solution) in proportions of 0.3 g per gram of crude reaction product was added to the product coming from the reactor to destroy the excess hypochlorite.

After reaching the steady state, the solution delivered contained 7.6% by weight of Gabapentin, corresponding to a yield of 93.3%.

### Example 2: Gabapentin synthesis in a reactor consisting of a series of agitated continuous reactors with a retention time of 20 minutes

A solution of 82.9 g of [1-(2-amino-2-oxoethyl)cyclohexyl]acetic acid in 167.0 g of 11% aqueous sodium hydroxide and a solution of 289.4 g of 13.4% aqueous sodium hypochlorite, with 32.7 g of 100% NaOH and 27.9 g of water for dilution were fed into the same reactor as in the previous example and maintained at 40°C, using flow rates of 1.67 ml/min and 1.8 ml/min, respectively, for a retention time of 19.5 minutes. Sodium metabisulphite (in 24% aqueous solution), in proportions of 0.1 g/gram of crude reaction product, was added to the product coming from the reactor to destroy the excess hypochlorite.

After reaching the stationary state, the solution delivered contained 11 % by weight of Gabapentin, corresponding to a yield of 88.1 %.

### Example 3: Gabapentin synthesis in a continuous tubular reactor with a retention time of 12 minutes.

A solution of 10.5 g of [1-(2-amino-2-oxoethyl)cyclohexyl]acetic acid in 23.2 g of 11 % aqueous sodium hydroxide and a solution of 302.7 g of 5% aqueous sodium hypochlorite, with 9.46 g of 100% NaOH were fed into a tubular reactor made of stainless steel AISI 316L with an internal diameter of 5 mm and a length of 240 mm, packed with polypropylene filler so as to leave a free volume of 4.8 ml, and maintained at 50°C. The flow rates were 0.116 g/min and 0.332 g/min, respectively, and the retention time was 12.5 minutes. Sodium metabisulphite (in 24% aqueous solution) in proportions of 0.3 g/gram of crude reaction product was added to the product coming from the reactor to destroy the excess hypochlorite. After reaching the stationary state, the solution delivered contained 5.9% by weight of Gabapentin, corresponding to a yield of 86%.

### Example 4: Gabapentin synthesis in a continuous tubular reactor with a retention time of 12 minutes

A solution of [1-(2-amino-2-oxoethyl)cyclohexyl]acetic acid in aqueous sodium hydroxide consisting of 1675 g of amide with a 52% humidity and 1153.3 g of 16.7% NaOH, and a solution of sodium hypochlorite and sodium hydroxide consisting of 3161.5 g of 13.4% aqueous hypochlorite, 332.6 g of 100%NaOH and 278.4 g of H₂O were fed into a glass reactor series consisting of a mixer with a volume of 25 ml, followed by a first tubular reactor with an internal diameter of 10 mm and a volume of 50 ml and then a second tubular reactor filled with glass beads, with a diameter of 3 cm and a free volume of 129 ml, and maintained at 40°C. The total volume of the reaction system, also considering the joints between the various components, was 249 ml. The flow rates were 420 ml/h and 634 ml/h, respectively, and the retention time was 14 minutes. Sodium metabisulphite (in 24% aqueous solution) in proportions of 0.11 g/gram of crude reaction product was added to the product coming from the reactor to destroy the excess hypochlorite, using a flow rate sufficient to adjust the potential of the solution to values below 100 mV with reference to an Ag/AgCl electrode.

After reaching the stationary state, the solution delivered contained 8.5% by weight of Gabapentin, corresponding to a yield of 96%.

### Example 5: Countercurrent liquid-liquid extraction in a Khuni column

A crude reaction product obtained using a continuous procedure in the system described in example 4 was adjusted to a pH=7 to obtain Gabapentin in zwitterionic form. The resulting solution, containing 6.52% by weight of Gabapentin, was fed into the top of a Khuni-type liquid-liquid extraction column with a diameter of 5 cm, comprising 23 agitated chambers, at a flow rate of 2 l/h. Butanol was fed into the bottom of the column at a flow rate of 3.8 l/h. From the top of the column, 4.2 l/h of butanol solution were drawn off, containing 4.35% by weight of Gabapentin, corresponding to a yield of 97.8%. The ash content, measured on the residue dried at 600°C, was 0.98%.

### Example 6: Countercurrent extraction and washing in a Khuni column

In the same liquid-liquid extraction column as described in Example 5, the crude reaction product obtained as in example 4, adjusted to a pH=7.2, and containing 6.52% of Gabapentin, was fed into the 8^{th} agitated chamber from the top at a flow rate of 1.02 l/h, while water was added at the top of the column at a flow rate of 0.6 l/h, and n-butanol was fed into the bottom of the column at a flow rate of 2 l/h. A 1.8 kg/h flow rate of the organic phase containing 2.4% of Gabapentin and 0.5% of ashes was obtained at the top of the column. The yield in Gabapentin amounted to 55%.

### Example 7: Extraction of the crude reaction product and washing of the butanol extract using a batch procedure

A quantity of 3.3 litres of the crude reaction product at a pH of 7.2, containing 7.49% of Gabapentin, and 2.2 litres of butanol were loaded in the reactor and left under vigorous stirring for 15 minutes at room temperature, then the mixture was allowed to decant for 15 minutes and the butanol phase was removed. The remaining aqueous phase was re-extracted, repeating the same procedure twice more. The combined organic phases, weighing a total of 6248 g, contained 3.9% of Gabapentin. The extraction yield was 82.6%.

The combined organic phases were washed with 2 L of demineralised water and, after phase separation, an organic phase was obtained with 2.82% of Gabapentin, corresponding to a yield from the crude reaction product of 64.3%.

### Example 8: Gabapentin precipitation from the butanol solution

The water content was removed azeotropically from the butanol solution coming from example 7, for a total amount of 6720 g, by distilling in a vacuum at 40°C, and the solution was then concentrated at a residual pressure of 15 mm to obtain a slurry containing 20% of Gabapentin. The slurry was cooled to 5°C, then filtered. The white solid, oven dried at 40°C under vacuum (15 mm) amounted to 190 g of Gabapentin with an assay of 90%, and 5.5% of NaCl; the yield from the organic phase was 89%.

### Example 9: Gabapentin crystallisation

100 g of 90% Gabapentin obtained as in example 8 were dissolved in 531 g of methanol and the mixture was boiled for 30 minutes. After hot filtering, the clear solution was left to cool naturally, bringing the temperature down to 5°C, and the resulting slurry was filtered, washing the panel on the filter twice with 100 ml of methanol. After drying at 40°C under vacuum, 63 g of Gabapentin with an assay of 98.5% were obtained, containing 280 ppm of chlorides, with a crystallisation yield of 70%.

## Claims

1. A method for the preparation of [(1-aminomethyl)cyclohexyl]acetic acid (Gabapentin) comprising at least the steps of:
- mixing and making to react in a continuous reactor an alkaline aqueous solution of [1-(2-amino-2-oxoethyl)cyclohexyl]acetic acid with an alkaline aqueous solution of a hypohalogenite;
- extracting Gabapentin in zwitterionic form from the previous reaction mixture with a higher aliphatic alcohol C₄-C₇, after neutralising the reaction mixture with an acid.

2. The method for the preparation of [(1-aminomethyl)cyclohexyl]acetic acid (Gabapentin) according to claim 1, comprising the further step of treating the reaction mass deriving from Hofmann re-arrangement with a reducing agent to reduce the excess hypohalogenite contained in the product coming from the reactor.

3. The method for the preparation of [(1-aminomethyl)cyclohexyl]acetic acid (Gabapentin) according to claim 1, wherein the hypohalogenite is sodium hypochlorite.

4. The method for the preparation of [(1-aminomethyl)cyclohexyl]acetic acid (Gabapentin) according to claim 1, wherein the alkali is sodium hydroxide.

5. The method for the preparation of [(1-aminomethyl)cyclohexyl]acetic acid (Gabapentin) according to claim 1, wherein the molar ratio between the hypohalogenite and the [1-(2-amino-2-oxoethyl)cyclohexyl]acetic acid is higher than 1.

6. The method for the preparation of [(1-aminomethyl)cyclohexyl]acetic acid (Gabapentin) according to claim 1, wherein the molar ratio between the alkali and the [1-(2-amino-2-oxoethyl)cyclohexyl]acetic acid is in the range comprised from 2 to 4.

7. The method for the preparation of [(1-aminomethyl)cyclohexyl]acetic acid (Gabapentin) according to claim 6, wherein the molar ratio between the alkali and the [1-(2-amino-2-oxoethyl)cyclohexyl]acetic acid is in the range comprised from 2.5 to 3.5.

8. The method for the preparation of [(1-aminomethyl)cyclohexyl]acetic acid (Gabapentin) according to claim 1, wherein the temperature of the continuous reactor is in the range comprised from 5 to 90°C.

9. The method for the preparation of [(1-aminomethyl)cyclohexyl]acetic acid (Gabapentin) according to claim 1, wherein the temperature of the continuous reactor is in the range comprised from 30°C to 50°C.

10. The method for the preparation of [(1-aminomethyl)cyclohexyl]acetic acid (Gabapentin) according to claim 1, wherein the retention time in the continuous reactor is in the range comprised from 2 minutes to 180 minutes.

11. The method for the preparation of [(1-aminomethyl)cyclohexyl]acetic acid (Gabapentin) according to claim 1, wherein the retention time in the continuous reactor is in the range comprised from 5 minutes to 60 minutes.

12. The method for the preparation of [(1-aminomethyl)cyclohexyl]acetic acid (Gabapentin) according to claim 1, wherein the extraction is liquid-liquid.

13. The method for the preparation of [(1-aminomethyl)cyclohexyl]acetic acid (Gabapentin) according to claim 12, wherein the extraction is in continuous mode.

14. The method for the preparation of [(1-aminomethyl)cyclohexyl]acetic acid (Gabapentin) according to claim 13, wherein the extraction is performed in counter-current mode.

15. The method for the preparation of [(1-aminomethyl)cyclohexyl]acetic acid (Gabapentin) according to claim 1, wherein the extraction solvent is an aliphatic alcohol C₄-C₇ selected from the group consisting of n-butyl and isobutyl alcohol, amyl and isoamyl alcohol, and mixtures thereof.

16. The method for the preparation of [(1-aminomethyl)cyclohexyl]acetic acid (Gabapentin) according to claim 13, wherein the extraction solvent is n-butanol.

17. The method for the preparation of [(1-aminomethyl)cyclohexyl]acetic acid (Gabapentin) according to claim 1, wherein the ratio between the flow rate of the alcohol and the flow rate of the neutralised reaction mixture in the extraction process is in the range of 0.5:1 to 3:1 v/v.

18. The method for the preparation of [(1-aminomethyl)cyclohexyl]acetic acid (Gabapentin) according to claim 1, wherein the water is removed from the extracted organic solution containing Gabapentin by azeotropic distillation.

19. The method for the preparation of [(1-aminomethyl)cyclohexyl]acetic acid (Gabapentin) by means of the Hofmann re-arrangement of [1-(2-amino-2-oxoethyl)cyclohexyl]acetic acid with hypohalogenite obtained in a continuous reactor.
